# EUROPÄISCHE PATENTSCHRIFT

(11) **EP 2 552 548 B1**
(45) Veröffentlichungstag und Bekanntmachung des Hinweises auf die Patenterteilung: **16.08.2017**
(21) Anmeldenummer: 11707812.1
(22) Anmeldetag: 25.02.2011
(51) Int. Cl.: A61Q 5/12

(54) **AUSSPÜLHAARBEHANDLUNGSMITTEL MIT NIEDRIG- BIS MITTELVISKOSEN POLYDIMETHYLSILOXANEN**
RINSE-OUT HAIR TREATMENT AGENT HAVING LOW- TO MEDIUM-VISCOSITY POLYDIMETHYLSILOXANES
PRODUIT TRAITANT DE RINÇAGE CAPILLAIRE CONTENANT DES POLYDIMÉTHYLSILOXANES DE VISCOSITÉ FAIBLE À MOYENNE

(30) Priorität: 31.03.2010 DE 102010013763
(43) Veröffentlichungstag der Anmeldung: 06.02.2013
(73) Patentinhaber: Beiersdorf AG, 20253 Hamburg (DE)
(72) Erfinder: SALADIN, Sandra, 20144 Hamburg (DE); KRAUSS, Nicole, Maria, 20148 Hamburg (DE)
(74) Vertreter: Hartmann, Jost
(86) Internationale Anmeldenummer: PCT/EP2011/052789
(87) Internationale Veröffentlichungsnummer: WO 2011/120742

(56) Entgegenhaltungen:
- EP-A1- 1 527 768
- EP-A1- 2 022 471
- EP-A1- 2 022 478
- WO-A1-03/092637
- WO-A2-2009/156663

## Beschreibung

Haarbehandlungsmittel werden zur Pflege nach der Reinigung der Haare - einer Form keratinischer Fasern- angewendet. Spülungen und Kuren werden normalerweise auf Basis von kationischen Tensiden wie beispielsweise Cetyl Trimethyl Ammonium Chlorid und Fettalkoholen wie Cetyl und/oder Stearylalkohol formuliert. Allein reicht diese Kombination jedoch nicht für eine geeignete konditionierende Pflege an Haaren wie unter anderem Geschmeidigkeit, Kämmbarkeit und Glanz aus. Aus diesem Grund werden häufiger Silikone wie beispielsweise langkettige Polydimethylsiloxane (PDMS) eingesetzt. Diese Silikone aus einer Spülung ermöglichen eine gute Verteilbarkeit, sowie auch ein seidiges Haargefühl im trockenen Zustand, jedoch bieten normalerweise eine mittlere Pflege an. Verbraucher, die widerspenstiges, trockenes und krisseliges Haar haben, wollen dieses Haar bändigen. Die Pflege von Spülungen mit PDMS reicht normalerweise nicht aus, um solche Haare unter Kontrolle zu bringen. Silikonhersteller wie Dow Corning bieten aminofunktionalisierte Silikone und hochmolekulare Dimethicone Gums an, die noch substantiver sind als PDMS (siehe beispielsweise http://www.dowcorning.com/content/personal/personalhair/default.asp), um eine intensive Pflegeleistung und Parallelisierung der Haarfaser für Haare, die zur Frizz neigen, zu gewährleisten. Nachteil liegt jedoch in der Neigung zur Beschwerung der Haare.

Die Schrift EP-1309649 B1 offenbart bestimmte Polysiloxan-Verbindungen mit Polyalkylenoxid-Struktureinheiten, mindestens einen zweiwertigen oder dreiwertigen organischen Rest, der mindestens eine Ammoniumgruppe enthält, mindestens eine Polysiloxan-Struktureinheit, mindestens einen organischen oder anorganischen Säurerest zur Neutralisation der aus der(n) Ammoniumgruppe(n) resultierenden Ladungen.

Die Schrift WO-2004069137 A2 offenbart die Verwendung mindestens eines bestimmten linearen oder verzweigten Polyamino-und/oder Polyammonium-Polysiloxancopolymer in der Herstellung und/oder Behandlung von gefärbtem Haar.

Die Schrift EP-1920758 A1 offenbart Reinigungszubereitungen für keratinische Fasern mit bestimmten Tensiden, C12-22 Monoglyceriden und Silikonpolymeren mit quternärer Ammoniumgruppe und Polyethergruppe.

EP 1527768 A1 offenbart die Kombination von einem Silicone Quat, Quaternium-80 und einem flüchtigen Silikonöl.

All dies konnte nicht den Weg zur vorliegenden Erfindung weisen. Es hat sich überraschend herausgestellt, dass eine Zubereitung zur Behandlung keratinischer Fasern enthaltend
a) ein kationisches Polysiloxan Polymer, auch bekannt als "Silicone Quat",
b) ein Dimethylsiloxan (PDMS) mit einer kinematischen Viskosität von 50cSt bis 5000 cSt,
c) gemessen mit Haake Viscotester VT 550 mit dem Messsystem NV bei 25°C,
d) ein kationisches Tensid, d) eine oder mehrere Fettverbindungen mit Schmelzpunkt > 35°C und einen wässrigen Träger den Nachteilen des Standes der Technik abhilft.

Überraschenderweise wurde gefunden, dass die Kombination von einem solchen PDMS niedriger bis mittlerer kinematischer Viskosität und einem Silicone Quat, besonders bevorzugt ein kationisches Polysiloxan Polymer mit quaternären Ammonium-Gruppen zu einer Parallelisierung der Haare führt ohne das Haar unnötig zu beschweren. Dieser Glättungseffekt zeigte sich an trocken/strapazierten 2 Std. gebleichten 2g Haarsträhnen von der Firma Kerling.

Die folgenden Handelsnamen beziehen sich auf das jeweils im Oktober 2008 auf dem Markt erhältliche Produkt. Es ist ganz besonders bevorzugt, wenn als kationisches Polysiloxan Polymer mit quaternären Ammonium-Gruppen Silicone Quaternium-18 mit den von Momentive angebotenen Handelsnamen Silsoft Q verwendet wird. Weitere bevorzugte kationisches Polysiloxan Polymere sind von Dow Corning unter dem Handelsnamen Dow Corning 5-7113 (Silicone Quaternium-16), Dow Corning 5-7070 (Silicone Quaternium-16/Glycidoxy Dimethicone Crosspolymer) bekannt, sowie auch Silquat Polyether Fatty Quats Typ Siltech AD (Silicone Quaternium-8), Silquat AC und Silquat D208 TEA von Siltech Corporation und Silicone Quaternium-22 bekannt als ABIL T Quat 60 von Evonik. Besonders bevorzugt ist es, wenn das kationisches Polysiloxan Polymer mit quaternären Ammonium-Gruppen in Konzentrationen von 0,25 bis 2,5 Gew.-%, bevorzugt von 0,5 bis 1,5 Gew.-%, jeweils bezogen auf den Aktivgehalt, eingesetzt wird. Besonders bevorzugt ist es weiterhin, wenn das Dimethylsiloxan PDMS in gleichen oder höheren Konzentrationen, als das kationische Polysiloxan Polymer mit quaternären Ammonium-Gruppen, jeweils bezogen auf den Aktivgehalt, eingesetzt wird, bevorzugt im Verhältnis Dimethylsiloxan PDMS zu kationischem Polysiloxan Polymer von 1.5:1, bis 2:1 und besonders bevorzugt ist 1.3:0.5.

Weiter ist es bevorzugt wenn ein PDMS als Fluid und nicht als Emulsion verwendet wird, bekannt als Xiameter® PMX-200 Silicone Fluid Serie von Dow Corning mit kinematischen Viskositäten von 50cSt bis maximal 5000cSt. Weitere Handelsnamen für Dimethicone sind unter Wacker Belsil DM Grades, BRB Silicone Oil DM Grades, SF96 (50-1000cs) Serie von Momentive Performance Materials und DM Fluid Serien (10cs - 3000cs) von Shin-Etsu Chemicals Co. bekannt. Ein weiteres bevorzugtes Silikonöl ist Baysilone-Oel M 100 von Momentive Performance Materials.

Vorzugsweise, ist eine Verwendung von Xiameter® PMX-200 Silicone Fluid zwischen 50cSt und 500cSt, besonders mit einer kinematischen Viskosität von 100cSt, Brechungsindex von 1,4030 und Oberflächenspannung von 20,9 dynes/cm.

Als langkettige Fettalkohole dienen C₁₄, C₁₆, C₁₈, C₂₂ Alkohole bekannt als Myristyl-, Cetyl-, Stearyl- und Behenyl Alkohole, beziehungsweise Kombinationen aus den vorhergenannten Fettalkoholen wie beispielsweise Cetearyl Alkohol. Die Fettalkohole sind in den erfindungsgemäßen Zusammensetzungen in Mengen von 5,0 bis 7,5 Gew.-%, vorzugsweise 6,5 bis 7,5 Gew.-%, bezogen auf die gesamte Zusammensetzung enthalten.

Es ist bekannt, dass kationische Amine, insbesondere tertiäre Amine in Kombination mit Fettalkoholen wie Cetyl und Stearyl Alkohol zu einer reichhaltigen, cremigen Spülungsgrundlage führen. Gemäß der Erfindung sind tertiäre Amine bekannt als Amidoamine besonders bevorzugte kationische Tenside in einem sauren Medium. Besonders bevorzugt ist die Verwendung von einem Amidoamin, bekannt als Stearamidopropyl Dimethylamine bekannt als Tego Amid S 18 von Evonik, sowie auch Behenamidopropyl Dimethylamine, bekannt als Amidet APA-22 von Kao. Diese Amidoamine werden in den erfindungsgemäßen Zusammensetzungen in einer Menge von 1,0 bis maximal 2,5%gew. eingesetzt.

Erfindungsgemäße Spülungen können auch Monoalkyltrimethylammoniumsalze mit einer Kettenlänge des Alkylrestes von 16 bis 24 Kohlenstoffatomen enthalten. Diese Verbindungen weisen die in der Formel (I) aus CTFA dargestellte Struktur auf, wobei R, R¹, R^{II}, und R^{III} für jeweils eine Methylgruppe stehen und R^{III} für einen gesättigten, verzweigten oder unverzweigten Alkylrest mit einer Kettenlänge von 16 bis 24 Kohlenstoffatomen und A- für ein Anion ausgewählt aus den physiologisch verträglichen Anionen ist.

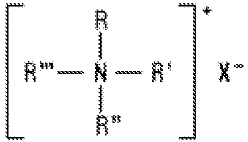

Beispielhaft für das Anion seien die Halogenide, Fluoride, Chloride, Bromide, Sulfate (Methosulfat) der allgemeinen Formel RSO₃⁻ worin R die Bedeutung von gesättigtem oder ungesättigtem Alkylresten mit 1 bis 4 Kohlenstoffatomen hat, oder anionische Reste organischer Säuren wie Malest, Fumarat, Oxalat, Tartrat, Citrat, Lactat oder Acetat, genannt.

Beispiele für Verbindungen der Formel (I) sind Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat. Bevorzugt sind Cetyltrimethylammonium- und Behenyltrimethylammoniumsalze. Besonders bevorzugt sind letztere in Form der Methosulfate, Chloride und Bromide. Am bevorzugtesten wird Cetyltrimethylammoniumchlorid und höchst bevorzugt ist Behenyltrimethylammoniumchlorid. Diese können in Kombination mit Amidoamin oder alleine eingesetzt werden. Besonders Vorteilhaft ist die Kombination von einem Amidoamin mit einer Monoalkyltrimethylammonium-Verbindung in einem Verhältnis von 1:1.

Die Verbindungen der Formel (I) werden in den erfindungsgemäßen Zusammensetzungen in einer Menge von 0,01 bis 5,0 Gew.-% verwendet. Bevorzugt werden 0,1 bis 2,5 Gew.-% verwendet. Besonders bevorzugt sind Mengen von 0,5 bis 4,0 Gew.-%. Die Mengenangaben beziehen sich jeweils auf die gesamte Zusammensetzung. Die Erfindung umfasst auch die Verwendung einer erfindungsgemäßen Zubereitung zur Glättung der Haare und der Frisur.

Erfindungsgemäß ist es von Vorteil, wenn zusätzlich 0,1 bis 1 Gew.-% Oryzanol enthalten sind. Weiter ist es erfindungsgemäß von Vorteil, wenn die Zubereitung frei von Cellulose Derivaten ist. Weiter ist es erfindungsgemäß von Vorteil, wenn die Zubereitung nicht waschaktiv ist. Weiter ist es erfindungsgemäß von Vorteil, wenn die Zubereitung frei von anionischen Tensiden ist.

Erfindungsgemäße Zubereitungen können zusätzlich UV- Filter, Polyole, Konservierungsmittel, hydrolysierte Proteine, Pflanzenextrakte, Parfümöle, und Antioxidantien sowie auch amphotere Tenside enthalten. Besonders vorteilhaft ist wenn Pflanzen-basierte Triglyceride besonders bevorzugt Baumwollsaat-, Avocado-, Mandel-, Argan-, oder Babassuöl und Pflanzen-basierte Ester, besonders bevorzugt Jojobaöl zusätzlich vorhanden sind. Auch vorteilhaft ist, wenn die erfindungsgemäße Zubereitung Keratinhydrolysat bekannt als Nutrilan Keratin W PP von Cognis enthält.

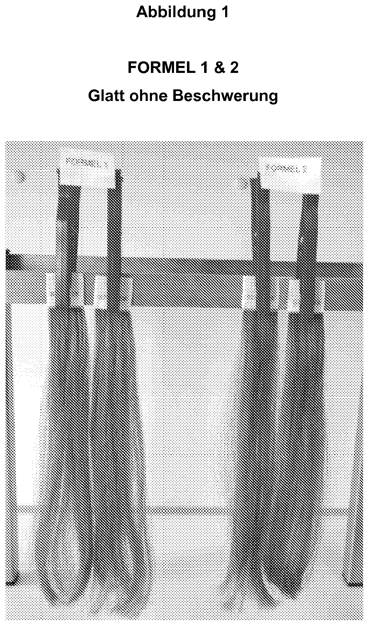

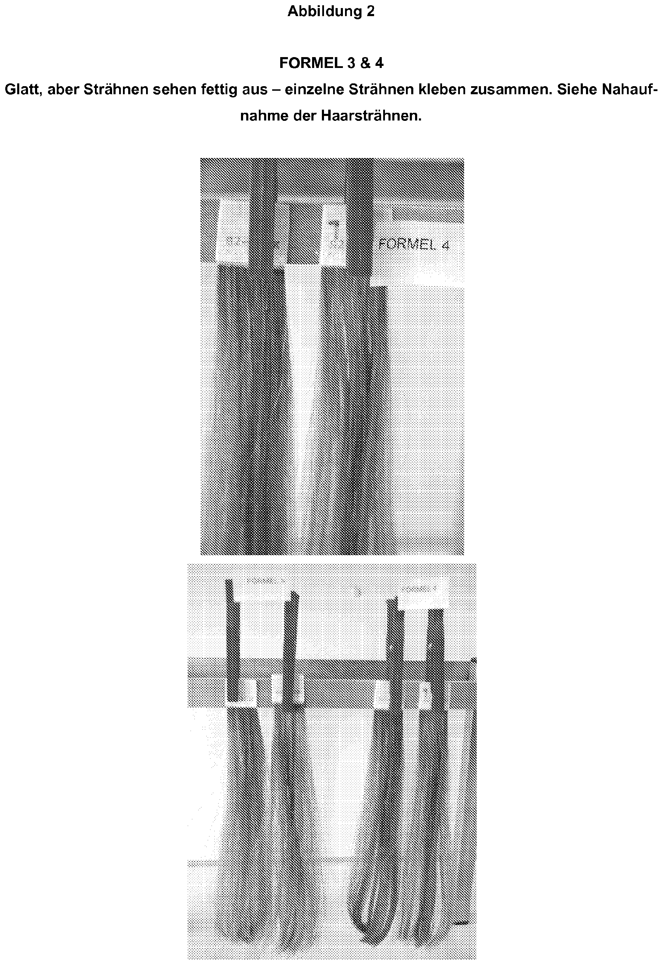

Behandlung einer Haarsträhne (2g 2 Std. gebleicht, 23cm lang; Firma Kerling) mit einer Tertiär-Amin basierten Spülung, 0,4mL (Formelbeispiele 1 & 2), die eine Kombination aus Xiameter PMX-200 Fluid 100CS und Silsoft Q (Silicone Quaternium-18) enthält, führte zu einem von einem Expertenpanel (n=5) sichtbar erkannten Glättungseffekt. Es wurde 1x und 3x mit der feinen Seite eines Kamms (Typ 623.7 Hercules Sägemann ® 394.7) gekämmt. In Bild 1 werden diese Strähnen abgebildet. Strähnen dagegen, die mit einem Dimethicone Fluid mit einer kinematischen Viskosität >5000cSt behandelt worden sind, zeigten zwar auch Parallelisierung jedoch mit dem Nachteil der Beschwerung (Formel 3). Auch DC 2-1491 Emulsion von Dow Corning in Kombination mit dem Silicone Quat zeigte ebenfalls diesen Nachteil (Formel 4).

| **Handelsnamen, Firma, Aktiv** | **inci** | **Bsp. 1** | **Bsp. 2** | **Bsp. 3** | **Bsp. 4** |
|---|---|---|---|---|---|
| Solbrol M, Lanxess, 99.5% | Methylparaben | 0,25 | 0,25 | 0,25 | 0,25 |
| Propylparaben, Schütz & Co., 99.5% | Propylparaben | 0,10 | 0,10 | 0,10 | 0,10 |
| Lanette-O, Cognis, 100% | Cetearyl Alcohol | | 6,80 | | |
| Nacol 16-95, Sasol, 95% | Cetyl Alcohol | 2,5 | | 2,5 | 2,5 |
| Baysilone-Oel M 100, Momentive (100%) Xiameter PMX-200 Silicone Fluid 100CS, Dow Corning (100%) | Dimethicone | 1,3 | 1,00 | | |
| Xiameter PMX-200 Silcone Fluid 30000CS, Dow Corning (100%) | Dimethicone | | | 1,3 | |
| MEM 1491 Emulsion, Dow Corning, 60% | Dimethicone,non-ionic emulsion of ultra high PDMS gum and PDMS fluid | | | | 2,2 |
| Lactic Acid, Biesterfeld, 90% | Lactic Acid | 0,80 | 0,40 | 0,80 | 0,80 |
| Sanal P, Akzo Nobel, 100% | Sodium Chloride | 0,01 | 0,1 | 0,01 | 0,01 |
| Lanette 18, Cognis, 100% | Stearyl Alcohol | 4,8 | | 4,8 | 4,8 |
| Dehyquart A-CA, Cognis, 25% Quat | Cetrimonium Chloride | | 4,00 | | |
| Gamma Oryzanol, Jan Dekker, 98% | Oryzanol | 0,01 | 0,01 | 0,01 | 0,01 |
| Dehyton AB 30, Cognis, 31% | Coco Betaine | 0,85 | 0,85 | 0,85 | 0,85 |
| Tego Amid S18, Evonik, 100% | Stearamidopropyl Dimethylamine | 2,2 | 1,00 | 2,2 | 2,2 |
| Silsoft Q, Momentive, 20% | Silicone Quaternium-18 + Trideceth-6 + Trideceth-12 | 2,5 | 2,5 | 2,5 | 2,5 |
| | Parfum | 0,70 | 0,70 | 0,70 | 0,70 |
| | Aqua | Ad. 100 | Ad. 100 | Ad. 100 | Ad. 100 |

### Protokoll fürs Expertenpanel:

Es werden 2g zwei Stunden gebleichte Haarsträhnen verwendet.

Die Haarsträhnen werden mindestens 15 min eingeweicht.

Die Hände werden mit Standardshampoo ohne Pflege gewaschen.

Die Haarsträhne wird entnommen und mit dem groben Kammsegment und danach mit dem feinen Kammsegment gekämmt.

Die Haarsträhne wird unter fließendem Wasser nass gemacht und ein Mal mit leichtem Druck ausgestrichen.

Eine Spritze wird mit 0,4 ml Produkt aufgezogen. Der Inhalt der Spritze wird auf die Haarsträhne aufgetragen.

Die Hände werden mit Wasser aus einem Pumpspender befeuchtet.

Das Produkt wird 60 s im angegebenen Takt (58bpm) von oben nach unten verstrichen und danach 30s einwirken gelassen. Anschließend wird das Produkt 50s lang ausgespült. Die Haarsträhne wird mit dem groben Kammsegment und danach mit dem feinen Kammsegment gekämmt.

Am Ende der Bewertung wird die Haarsträhne wieder nass gemacht und die ganze Prozedur wiederholt. Die Produkte werden nach einer Doppelbehandlung bewertet, um Unterschiede klarer darzustellen.

Die folgenden Beispiele sollen den Gegenstand der vorliegenden Erfindung weiter erläutern.

| **Handelsnamen, Firma, Aktiv** | **inci** | **5** | **6** | **7** | **8** | **9** | **10** | **11** | **12** |
|---|---|---|---|---|---|---|---|---|---|
| Solbrol M, Lanxess, 99.5% | Methylparaben | 0,25 | 0,25 | 0,25 | 0,25 | 0,25 | 0,25 | 0,25 | 0,25 |
| Propylparaben, Schütz & Co.,99.5% | Propylparaben | 0,10 | 0,10 | 0,10 | 0,10 | 0,10 | 0,10 | 0,10 | 0,10 |
| Citronensäure, DSM, 100% | Citric Acid | | | | | | 0,01 | 0,01 | |
| Lanette-O, Cognis, 100% | Cetearyl Alcohol | | | | | 6,30 | 3,50 | 4,44 | |
| Nacol 16-95, Sasol, 95% | Cetyl Alcohol | 2,5 | 2,5 | 2,5 | 2,5 | | | | 2,5 |
| Pricerine 9086, Croda, 99% | Glycerin | | | | | | | 5,00 | |
| Natronlauge, Brenntag Chemiepartner,45% | Sodium Hydroxide | | | | | | 0,02 5 | 0,02 5 | |
| Baysilone-Oel M 100, Momentive, 100% Xiameter PMX-200 Silicone Fluid 100CS, Dow Corning 100% | Dimethicone | 1,5 | | | | 1,00 | 1,00 | 1,5 | 1,3 |
| Xiameter PMX-200 Silicone Fluid 350cSt, Dow Corning, 100% | | | 1,3 | | | | | | |
| Xiameter PMX-200 Silicone Fluid 500CS, Dow Corning 100% | Dimethicone | | | 1,3 | | | | | |
| Xiameter PMX-200 Silicone Fluid 1000CS, Dow Corning 100% | | | | | 1,5 | | | | |
| Dehyquart F 75, Cognis, 69,935% Quat | Distearoylethyl Hydroxyethylmonium Methosulfate + Cetearyl Alcohol | | | | | | | 2,86 | |
| Varisoft PATC, Evonik, 60% Quat | Palmitamidopropyltr imonium Chloride + Propylene Glycol | | | | | | | 1,66 | |
| Lactic Acid 90%, Biesterfeld | Lactic Acid | 0,80 | 0,80 | 0,80 | 0,80 | 0,40 | | | 0,80 |
| | Sodium Chloride | 0,01 | 0,01 | 0,01 | 0,01 | | | | 0,01 |
| Lanette 18, Cognis, 100% | Stearyl Alcohol | 4,8 | 4,8 | 4,8 | 4,8 | | | | 4,8 |
| Incroquat Behenyl TMC-25, Croda, 25% Quat | Behentrimonium Chloride + Cetearyl Alcohol | | | | | 0,2 | 0,2 | | |
| Dehyquart A-CA, Cognis, 25% Quat | Cetrimonium Chloride | | | | | | 2,00 | | |
| Gamma Oryzanol, Jan Dekker, 98% | Oryzanol | 0,01 | 0,01 | 0,01 | 0,01 | 0,01 | 0,01 | 0,05 | 0,01 |
| Dehyton AB 30, Cognis, 31% | Coco Betaine | 0,85 | 0,85 | 0,85 | 0,85 | 0,85 | | | 0,85 |
| Tego Amid S18, Evonik, 100% | Stearamidopropyl Dimethylamine | 2,2 | 2,2 | 2,2 | 2,2 | 1,00 | | | 2,2 |
| Silsoft Q, Momentive, 20% | Silicone Quaternium-18 + Trideceth-6 + Trideceth-12 | 1,5 | 2,5 | 2,5 | 2,0 | 2,5 | 2,5 | 2,5 | |
| DC 5-7113 Silicone Quat Microemulsion, 22% | Silicone Quaternium-16 + Undeceth-11 + Butyloctanol + Undeceth-5 | | | | | | | | 2,3 |
| | Parfum | 0,70 | 0,70 | 0,70 | 0,70 | 0,70 | 0,70 | 0,70 | 0,70 |
| | Aqua | Ad. 100 | Ad. 100 | Ad. 100 | Ad. 100 | Ad. 100 | Ad. 100 | Ad. 100 | Ad. 100 |

## Patentansprüche

1. Zubereitung zur Behandlung keratinischer Fasern enthaltend
a. ein kationisches Polysiloxan Polymer mit quaternären Ammonium-Gruppen,
b. ein Dimethylsiloxan mit einer kinematischen Viskosität von bis 50cSt bis 5000 cSt, gemessen mit Haake Viscotester VT 550 mit dem Messsystem NV bei 25°C,
c. ein kationisches Tensid,
d. eine oder mehrere Fettverbindungen mit Schmelzpunkt > 35°C
und einen wässrigen Träger.

2. Zubereitung nach einem der vorangehenden Ansprüche **dadurch gekennzeichnet, dass** als kationisches Polysiloxan Polymer Silicone Quaternium-18, Silicone Quaternium-8, Silicone Quaternium-16, Silicone Quaternium-16/Glycidoxy Dimethicone Crosspolymer, Silicone Quaternium-22 verwendet werden.

3. Zubereitung nach einem der vorangehenden Ansprüche **dadurch gekennzeichnet dass** als Dimethylsiloxan ein nicht emulsionsförmiges Fluid verwendet wird, das Xiameter® PMX-200 Silicone Fluid Serie von Dow Corning mit kinematischen Viskositäten von 50cSt bis maximal 5000cSt verwendet wird.

4. Zubereitung nach einem der vorangehenden Ansprüche **dadurch gekennzeichnet dass** als kationisches Tensid bevorzugt Stearamidopropyl Dimethylamine oder Behenamidopropyl Dimethylamine verwendet wird.

5. Zubereitung nach einem der vorangehenden Ansprüche **dadurch gekennzeichnet dass** das kationischem Polysiloxan Polymer mit quaternären Ammonium-Gruppen in Konzentrationen von 0,25 bis 2,5 Gew.-%, bevorzugt von 0,5 bis 1,5 Gew.-%, jeweils bezogen auf den Aktivgehalt, eingesetzt wird.

6. Zubereitung nach Anspruch 5 **dadurch gekennzeichnet, dass** das Polydimethylsiloxan in gleichen oder höheren Konzentrationen, als das kationische Polysiloxan Polymer mit quaternären Ammonium-Gruppen, jeweils bezogen auf den Aktivgehalt, eingesetzt wird, bevorzugt im Verhältnis Polydimethylsiloxan zu kationisches Polysiloxan Polymer von 1,5 : 1 bis 2 :1 und bevorzugt 1,3 : 0,5.

7. Verwendung einer Zubereitung nach einem der vorangehenden Ansprüche zur Glättung der Haare und der Frisur.

## Claims

1. Preparation for treating keratin fibres comprising
a. a cationic polysiloxane polymer having quaternary ammonium groups,
b. a dimethylsiloxane having a kinematic viscosity of 50 cSt to 5000 cSt, measured with a Haake Viscotester VT 550 with the NV measuring system at. 25°C,
c. a cationic surfactant,
d. one or more fatty compounds having a melting point >35°C
and an aqueous carrier.

2. Preparation according to any of the preceding claims, **characterized in that** the cationic polysiloxane polymer used is silicone quaternium-18, silicone quaternium-8, silicone quaternium-16, silicone quaternium-16/glycidoxy dimethicone crosspolymer, silicone quaternium-22.

3. Preparation according to either of the preceding claims, **characterized in that** the dimethylsiloxane used is a non-emulsion-style fluid, the Xiameter® PMX-200 silicone fluid series from Dow Corning being used having kinematic viscosities of 50 cSt up to a maximum of 5000 cSt.

4. Preparation according to any of the preceding claims, **characterized in that** the cationic surfactant used is preferably stearamidopropyl dimethylamine or behenamidopropyl dimethylamine.

5. Preparation according to any of the preceding claims, **characterized in that** the cationic polysiloxane polymer having quaternary ammonium groups is used at concentrations of 0.25 to 2.5% by weight, preferably from 0.5 to 1.5% by weight, based in each case on the active content.

6. Preparation according to Claim 5, **characterized in that** the polydimethylsiloxane is used at the same or higher concentrations than the cationic polysiloxane polymer having quaternary ammonium groups, based in each case on the active content, preferably in a ratio of polydimethylsiloxane to cationic polysiloxane polymer of from 1.5:1 to 2:1 and preferably 1.3:0.5.

7. Use of a preparation according to any of the preceding claims for smoothing hair and hairstyling.

## Revendications

1. Préparation pour le traitement de fibres kératiniques, contenant :
a. un polymère de polysiloxane cationique contenant des groupes ammonium quaternaires,
b. un diméthylsiloxane ayant une viscosité cinématique de 50 cSt à 5 000 cSt, mesurée avec un Haake Viscotester VT 550 avec le système de mesure NV à 25 °C,
c. un tensioactif cationique,
d. un ou plusieurs composés gras ayant un point de fusion > 35 °C,
et un véhicule aqueux.

2. Préparation selon l'une quelconque des revendications précédentes, **caractérisée en ce que** le silicone quaternium-18, le silicone quaternium-8, le silicone quaternium-16, le silicone quaternium-16/glycidoxy dimethicone crosspolymer, le silicone quaternium-22 sont utilisés en tant que polymère de polysiloxane cationique.

3. Préparation selon l'une quelconque des revendications précédentes, **caractérisée en ce qu'**un fluide non sous forme d'émulsion est utilisé en tant que diméthylsiloxane; le Xiameter® PMX-200 Silicone Fluid Serie de Dow Corning ayant des viscosités cinématiques de 50 cSt à au plus 5 000 cSt étant utilisé.

4. Préparation selon l'une quelconque des revendications précédentes, **caractérisée en ce que** la stéaramidopropyldiméthylamine ou la béhénamidopropyldiméthylamine est utilisée de préférence en tant que tensioactif cationique.

5. Préparation selon l'une quelconque des revendications précédentes, **caractérisée en ce que** le polymère de polysiloxane cationique contenant des groupes ammonium quaternaires est utilisé en concentrations de 0,25 à 2,5 % en poids, de préférence de 0,5 à 1,5 % en poids, à chaque fois par rapport à la teneur en agent actif.

6. Préparation selon la revendication 5, **caractérisée en ce que** le polydiméthylsiloxane est utilisé en des concentrations identiques ou supérieures au polymère de polysiloxane cationique contenant des groupes ammonium quaternaires, à chaque fois par rapport à la teneur en agent actif, de préférence en un rapport polydiméthylsiloxane sur polymère de polysiloxane cationique de 1,5:1 à 2:1, et de préférence de 1,3:0,5.

7. Utilisation d'une préparation selon l'une quelconque des revendications précédentes pour lisser les cheveux et les coiffures.
